# EUROPÄISCHE PATENTSCHRIFT

(11) **EP 2 121 024 B1**
(45) Veröffentlichungstag und Bekanntmachung des Hinweises auf die Patenterteilung: **28.10.2015**
(21) Anmeldenummer: 08716334.1
(22) Anmeldetag: 07.03.2008
(51) Int. Cl.: A61K 47/10, A61K 31/714, A61K 31/66, A61K 31/662

(54) **STABILISIERUNG VON VITAMIN B12**
STABILIZATION OF VITAMIN B12
STABILISATION DE VITAMINE B12

(30) Priorität: 16.03.2007 DE 102007012644
(43) Veröffentlichungstag der Anmeldung: 25.11.2009
(73) Patentinhaber: Bayer Intellectual Property GmbH, 40789 Monheim (DE)
(72) Erfinder: HEEP, Iris, 50859 Köln (DE); TATERRA, Hans-Rolf, 51519 Odenthal (DE)
(74) Vertreter: BIP Patents
(86) Internationale Anmeldenummer: PCT/EP2008/001819
(87) Internationale Veröffentlichungsnummer: WO 2008/113483

(56) Entgegenhaltungen:
- WO-A-2005/002589
- WO-A-2005/094842
- DE-C- 951 162

## Beschreibung

Die Erfindung betrifft Zubereitungen enthaltend Vitamin B₁₂ und ein Butanol sowie die Verwendung von Butanol zur Stabilisierung von Vitaminen.

Dass Vitamine nicht sehr stabil sind und z. B. bei Lagerung ein Abbau zu beobachten ist, ist allgemein bekannt. Dies gilt auch für die sogenannten B-Vitamine, explizit das Vitamin B₁₂. Daher gibt es sehr viele Arbeiten zu dem Thema der Stabilisierung von Vitaminen bzw. von Vitamin B₁₂. Eine einfache Lösung dieser Problematik ist z.B. das Separieren des Vitamins B₁₂ von Substanzen, die dessen Abbau fördern. So werden z.B. in JP200612486 3 Teillösungen beschrieben, in denen einzelne Teilformulierungen vorliegen. Nicht nur in flüssigen Formulierungen, auch in Tabletten oder Granulaten wird Vitamin B₁₂ stabilisiert, wie z.B. in EP416773 beschrieben ist. Der größte Anteil der Arbeiten zur Stabilisierung von Vitamin B₁₂ fällt allerdings auf flüssige Formulierungen. Solche Lösungen können auch in Kapseln (FR1472901) gefüllt werden oder die Lösungen werden zur weiteren Stabilisierung gefriergetrocknet (JP63313736). Unter den gebrauchsfertigen Lösungen von Vitamin B₁₂ gibt es einige, die durch den Zusatz von Eisensalzen stabilisiert werden (FR1285213, GB902377). In den Lösungen kann ebenfalls EDTA (Ethylendiamintetraessigsäure, US2939821 oder GB822127) enthalten sein. Auch Alkalisalze oder Erdalkalisalze sollen auf Vitamin B₁₂ stabilisierend wirken (US2566123). Vitamine können auch durch Verwendung von Aminosäuren (US2748054) oder durch Cyclodextrine (JP4049239), wie auch durch Ammoniumsulfat (US2778771), durch Natriumiodid (JP41007474), Kaliumcyanid (GB692968), Maleinsäure (JP64011864), Thiopropionsäure (US2579679), Gluconolacton (ES247522), Lecithine (JF55049313), Harnstoff (JP43010862) oder auch durch Fettsäureester mit Cystein (US2662048) oder durch Antioxidantien (DE25222187) und sogenannte Chelatbildner (WO97/31620) erfolgen. Sogar Lösungen in N-Methylpyrrolidon DE3337304 sind beschrieben, in denen das Vitamin B₁₂ stabil sein soll. In einigen Arbeiten wird Vitamin B₁₂ durch den Einsatz von polyvalenten Alkoholen stabilisiert (JP2311417, JP63313736, JP05124967, FR1263794, JP04-235925, JP2000-319186 oder HU150885). Allgemein wird die Verwendung von Alkoholen zur Stabilisierung von Vitamin B₁₂ in JP2005247800, WO2005/094842, WO02/02145, BE576619 oder JP02145521 vorgeschlagen. In US2005074443 sind langkettige Alkohole zur Stabilisierung von Vitamin B₁₂ beschrieben. Aber auch das Vitamin B₁₂ selbst wird verwendet, um wiederum andere Substanzen zu stabilisieren (JP2001-048780, JP2005-015368).

So wird in JP46-15320 Propylenglykol zur Isotonisierung und Benzylalkohol als Analgetikum eingesetzt. Die eigentliche Stabilisierung wird durch Dextran und Gelatine (hydrophile Makromoleküle) erreicht. In JP45-011919 werden die Alkohole Propylenglykol, Benzylalkohol, Mannitol oder Glycerol zur Stabilisierung von *5,6-dimethylbenzimidazolyl cobamide coenzyme* beschrieben.

Es wurde eine Möglichkeit gefunden, Vitamin B₁₂ durch den Einsatz von Butanol zu stabilisieren. In JP2005247800 sind zwar Alkohole zur Stabilisierung von Vitamin B₁₂ genannt, z.B. ist aber das dort enthaltene Chlorbutanol gerade für die in diesem Fall verwendete Lösung nicht geeignet, sondern schädlich. Ebenso ist der in JP46-15320 oder JP45-011919 enthaltene Benzylalkohol für die hier beschriebene Formulierung deutlich von Nachteil, da Benzylalkohol nicht von allen Tieren verstoffwechselt werden kann (EMEA, Committee for veterinary medicinal products, summary report, benzyl alcohol, 1997) und Formulierungen mit Benzylalkohol daher nicht so breit einsetzbar sind wie Formulierungen mit Butanol.

Die Erfindung betrifft:
- Eine Zubereitung enthaltend Vitamin B₁₂ und Butanol.
- Die Verwendung einer solchen Zubereitung zur Herstellung eines Arzneimittels.
- Die Verwendung von Butanol zur Stabilisierung von Vitamin B₁₂.
- Die Verwendung von Butanol zur Herstellung von Zubereitungen des Vitamin B₁₂ mit verbesserter Vitamin B₁₂-Stabilität

Unter Vitamin B₁₂ im engeren Sinne wird häufig Cyanocobalamin verstanden, es gibt jedoch auch andere Verbindungen die ebenfalls unter den Oberbegriff Vitamin B₁₂ fallen; diese werden auch als Cobalamine bezeichnet. Hier sollen unter dem Begriff Vitamin B₁₂ allgemein alle Verbindungen verstanden werden, die im menschlichen und/oder tierischen Körper als Coenzym wirken oder in die entsprechenden Coenzymformen umgewandelt werden können. Gemeinsam ist diesen Vitamin B₁₂-Verbindungen das Corrin-Gerüst mit einem dreiwertigen Cobalt als Zentralatom und einem über D-Ribofuranose-3-phosphat alpha-glykosidisch gebundenen 5,5-Dimethylbenzimidazol-Rest. Die meisten Cobalamine unterscheiden sich lediglich in einem axialen Substituenten voneinander. Beispiele für Verbindungen, die als Vitamin B₁₂ eingesetzt werden können, sind: Cyanocobalamin (axialer Substituent = CN), Aquocobalamin (B₁₂ₐ, axialer Substituent = -O⁺H₂), Hydroxocobalamin (B_{12b}, axialer Substituent = -OH), Nitritocobalamin (B_{12c}, axialer Substituent = -NO₂), 5'-Desoxyadenosylcobalamin (Coenzym B₁₂, axialer Substituent = 5'-Desoxyadenosyl) und Methylcobalamin (Methyl B₁₂, axialer Substituent = -CH₃). Adenosylcobalamin und Methylcobalamin sind die eigentlichen Wirkformen (Coenzymformen), Aquocobalamin und Hydroxocobalamin sind Speicherformen, die ebenfalls im Körper vorkommen.

Gemäß einer bevorzugten Ausführungsform enthalten die erfindungsgemäßen Zubereitungen zusätzlich ein pharmakologisch verträgliches Phosphonsäurederivat. Erfindungsgemäß einsetzbare pharmakologisch verträgliche Phosphonsäurederivate sind üblicherweise organische Verbindungen, die sich als Stoffwechselstimulantien und Tonika insbesondere für Nutz- und Haustiere eignen. Als bevorzugte Beispiele seien die bereits lange bekannten Verbindungen Butaphosphan und Toldimfos genannt, die unter anderem der Ergänzung von Mineralstoffen (Phosphor) dienen.

Butaphosphan trägt die chemische Bezeichnung (1-Butylamino-1-methyl)ethylphosphonsäure und hat die Strukturformel

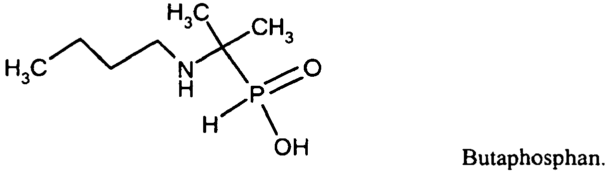

Butaphosphan wird üblicherweise als freie Säure eingesetzt.

Toldimfos trägt die chemische Bezeichnung (4-Dimethylamino-o-tolyl)phosphonsäure und hat die folgende Strukturformel

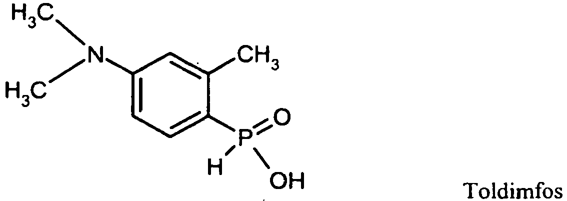

Toldimfos wird üblicherweise als Natriumsalz eingesetzt.

Von der vorliegenden Erfindung umfasst wird sowohl die Verwendung der freien Wirkstoffe als auch ihrer pharmazeutisch akzeptablen Salze sowie die Verwendung der entsprechenden Hydrate und Solvate der Verbindungen oder ihrer Salze.

In den erfindungsgemäßen Arzneimitteln wird das Vitamin B₁₂ typischerweise in einem Anteil von 0,00001 bis 0,1 %, bevorzugt 0,0001 bis 0,05 % und besonders bevorzugt mit 0,001 bis 0,01 % eingesetzt. Hier und im Folgenden sind - soweit nicht anders angegeben - unter den Prozentangaben Prozent (M/V) zu verstehen. Dies bedeutet: Masse der betreffenden Substanz in Gramm pro 100 ml fertiger Lösung.

Butanol bezeichnet isomere aliphatische Alkohole, die eine Alkylkette mit vier Kohlenstoffatomen aufweisen, sie können linear oder verzweigt vorliegen und zwar als n-Butanol, sek.-Butanol, tert.-Butanol und iso-Butanol. Bevorzugt sind iso-Butanol und n-Butanol und insbesondere n-Butanol.

Butanol wird üblicherweise in Konzentrationen von 0,1 bis 10 %, bevorzugt von 0,5 bis 7 % und besonders bevorzugt von 1 bis 5 % eingesetzt. Die Prozentangaben bedeuten % (M/V).

In den erfindungsgemäßen Arzneimitteln werden die pharmakologisch wirksamen Phosphonsäuren, wie z. B. Toldimfos oder insbesondere Butaphosphan, in einem Anteil von 0,1 bis 40 %, bevorzugt 1 bis 30 % und besonders bevorzugt mit 5 bis 20 % eingesetzt.

Die erfindungsgemäßen Zubereitungen sind vorzugsweise flüssig und enthalten üblicherweise Wasser oder eine wassermischbare Substanz als Solvens. Als Beispiele seien genannt Glycerin, Propylenglykol, Polyethylenglykole, verträgliche Alkohole wie Ethanol oder Benzylalkohol, N-Methylpyrrolidon, Propylencarbonat, Glycofurol, Dimethylacetamid, 2-Pyrrolidon, Isopropylidenglycerol, oder Glycerinformal. Die Solventien können auch in Mischungen oder Kombinationen eingesetzt werden. Bevorzugt sind Formulierungen auf Wasserbasis, in denen selbstverständlich weitere Solventien und Kosolventien enthalten sein können.

Als Solvens kann die flüssige Formulierung außer Wasser oder wassermischbaren Substanzen auch Öle in Form einer Emulsion enthalten. Hierunter seien die pflanzlichen, tierischen und synthetischen Öle wie Baumwollsamenöl, Sesamöl, Sojaöl, Mittelkettige Triglyceride einer Kettenlänge von C₁₂-C₁₈, Propylenglykoloctanoat-decanoat oder auch Paraffin genannt.

Das Solvens (oder das Solvensgemisch) ist üblicherweise in Konzentrationen von bis zu 98 %, bevorzugt bis zu 90 %, besonders bevorzugt bis zu 87 % enthalten. In der Regel liegen die Konzentrationen des Solvens über 65 %, bevorzugt über 75 %, besonders bevorzugt über 85 %. Die Prozentangaben bedeuten % (M/V).

Die erfindungsgemäßen Formulierungen können auch Kosolventien enthalten, und zwar bevorzugt dann, wenn die Formulierungen Wasser enthalten; die Kosolventien können die Löslichkeit bestimmter Formulierungsbestandteile verbessern. Die Kosolventien werden üblicherweise in Anteilen von 0,1 bis 30 % bevorzugt von 1 bis 10 % eingesetzt (Prozentangaben jeweils M/V). Als Kosolventien seien beispielsweise genannt: Pharmazeutisch verträgliche Alkohole, Dimethylsulfoxid, Ethyllactat, Ethylacetat, Triacetin. Auch Mischungen der vorstehend genannten Lösungsmittel können als Kosolvens eingesetzt werden. Unter Umständen können einzelne Kosolventien auch als Solventien eingesetzt werden. Üblicherweise werden in den erfindungsgemäßen Zubereitungen nur solche Kosolventien eingesetzt, die nicht schon als Solvens oder im Solvensgemisch verwendet wurden.

Konservierungsmittel können in der flüssigen Formulierung enthalten sein. Als Beispiele für verwendbare Konservierungsmittel seien genannt: Aliphatische Alkohole wie Benzylalkohol, Ethanol, n-Butanol, Phenol, Cresole, Chlorbutanol, para-Hydroxybenzoesäureester (insbesondere die Methyl- und Propylester), Salze oder die freien Säuren der Carbonsäuren, wie Sorbinsäure, Benzoesäure, Milchsäure oder Propionsäure. Ebenso die quartären Ammoniumverbindungen, wie z. B. Benzalkoniumchlorid, Benzethoniumchlorid oder Cetylpyridiniumchlorid. Konservierungsmittel werden üblicherweise in Anteilen von 0,001 bis 5 % bevorzugt von 0,01 bis 4 % eingesetzt. Sofern die erfindungsgemäßen Zubereitungen bereits in ausreichender Menge eine Komponente enthalten, die als Konservierungsmittel dienen kann (z. B. n-Butanol), wird in der Regel kein zusätzlicher Anteil mehr zu Konservierungzwecken zugesetzt. Allerdings können gegebenenfalls noch andere Konservierungsmittel zugegeben werden.

Je nach Art der Formulierung und Applikationsform können die erfindungsgemäßen Arzneimittel weitere übliche, pharmazeutisch verträgliche Zusatz- und Hilfsstoffe enthalten. Als Beispiele seien genannt
- Antioxidantien wie zum Beispiel Sulfite (Na-sulfit, Na-metabisulfit), organische Sulfide (Cystin, Cystein, Cysteamin, Methionin, Thioglycerol, Thioglykolsäure, Thiomilchsäure) Phenole (Tocopherole, wie auch Vitamin E und Derivate davon z.B. Vitamin-E-TPGS (d-alpha-Tocopherylpolyethylenglykol-1000-succinat)), Butylhydroxyanisol, Butylhydroxytoluol, Octyl- und Dodecylgallat), organische Säuren (Ascorbinsäure, Citronensäure, Weinsäure, Milchsäure) und deren Salze und Ester. Üblicherweise werden 0,01-5, bevorzugt 0,05-1 % eingesetzt.
- Netzmittel wie zum Beispiel Fettsäuresalze, Fettalkylsulfate, Fettalkylsulfonate, lineare Alkylbenzolsulfonate, Fettalkylpolyethylen-glykolethersulfate, Fettalkylpolyethylenglykolether, Alkylphenolpolyethylenglykolether, Alkylpolyglykoside, Fettsäure-N-methylglucamide, Polysorbate, Sorbitanfettsäureester und Poloxamere. Üblicherweise werden 0,01-10%, bevorzugt 0,1 -5 % eingesetzt.
- Substanzen zur Isotonisierung wie z.B. Natriumchlorid, Glucose oder Glycerin. Üblicherweise werden 0,01-5 %, bevorzugt 0,1-1 % eingesetzt.
- Substanzen die eine Partikelbildung vermeiden können; z.B. Poloxamere, Lecithine, Polyvinylpyrrolidone, Kosolventien, Antioxidantien wie z.B. Natriumdisulfit oder Komplexbildner wie z.B. das Natriumsalz der Editinsäure. Üblicherweise werden 0,01-5 %, bevorzugt 0,05-1 % eingesetzt.
- Die flüssigen Formulierungen können Substanzen enthalten, welche die lokale Verträglichkeit bei Applikation verbessern. Als Beispiele seien genannt: Radikalfänger oder Antioxidantien wie z.B. Vitamin E, oder Vitamin C, Butylhydroxyanisol, Butylhydroxytoluol, Cysteamin, Cystein, Glutathion, Thioglykol, Thiomilchsäure, Natriumdisulfid oder auch Acetylcystein. Komplexbildner wie z.B. Cyclodextrine (z. B. Hydroxyproypl-Cyclodextrin), Natrium-EDTA (Ethylendiamintetraessigsäure), Polyvinlypyrrolidon, Dexpanthenol, Salze von Fettsäuren wie z.B. Natriumcaprylat, Salze mehrwertiger Metallkationen (z. B. Me²⁺ bzw. Me³⁺), insbesondere der Erdalkalimetalle, Aminosäuren und hierunter besonders Arginin oder Lysin, Poloxamere, Poloxamine, Solventien oder Kosolventien wie z.B. Glycerin, Polyethylenglykol, Propylenglykol oder Dimethylacetamid, Dextrane, Kreatin, Kreatinin, Säuren wie z.B. die Gluconolactonsäure, Milchsäure, Embonsäure, Zitronensäure, Weinsäure, Schleimsäure oder Hyaluronsäure, Lecithine mit einem Gehalt an Phosphatidylcholin von 70-100 % aus Soja oder Hühnereiweiß. Üblicherweise werden 0,01-20 %, bevorzugt 0,1-10 % eingesetzt.
- Pharmazeutisch akzeptable Farbstoffe wie zum Beispiel Eisenoxide, Carotinoide, etc. Üblicherweise werden 0,01-10 %, bevorzugt 0,1-5 % eingesetzt.

Der pH-Wert der flüssigen Zubereitungen beträgt 2-11, bevorzugt 3-8 und besonders bevorzugt 4-7. Der pH-Wert wird gegebenenfalls durch Zugabe pharmazeutisch annehmbarer Säuren oder Basen eingestellt. Falls die Zubereitungen eine pharmakologisch wirksame Phosphonsäure enthalten, wird vorzugsweise eine Base zur Einstellung der oben angegebenen pH-Werte zugegeben.

Als Basen können beispielsweise verwendet werden: Alkali- oder Erdalkalihydroxide (z.B. NaOH, KOH; gegebenenfalls in Form ihrer wässrigen Lösungen: Natronlauge, Kalilauge), oder basische Phosphate z. B. Natriumphosphat, Natriumhydrogenphosphat, basische Aminosäuren wie z.B. Lysin, Arginin, Histidin, Ornithin, Citrullin, Hydroxylysin, Cholin, Meglumin, Ethanolamine wie Triethanolamin oder auch Puffer (Tris(hydroxymethyl)aminomethan, Cyclohexylamino-1-Propansulfonsäure). Bevorzugt werden als Base eingesetzt: NaOH, KOH oder Arginin; besonders bevorzugt ist NaOH, gegebenenfalls als wässrige Natronlauge.

Als Säuren können beispielsweise verwendet werden: Anorganische Säuren wie z. B. Salzsäure, Schwefelsäure, Phosphorsäure oder organische Säuren, wie z. B. Methansulfonsäure, Ameisensäure, Essigsäure, Propionsäure, Milchsäure, Malonsäure, Adipinsäure, Weinsäure, Oxalsäure, Fumarsäure, Äpfelsäure, Citronensäure, Bernsteinsäure, Asparaginsäure, Glutaminsäure, Gluconsäure, Glucuronsäure, Galacturonsäure, Glutarsäure, Lactobionsäure, Mandelsäure, Salicylsäure, Ascorbinsäure, Benzoesäure, Maleinsäure, Zitronensäure, Octansäure, Linolsäure, Linolensäure.

Die benötigte Menge an Säure oder Base ergibt sich aus dem angestrebten pH-Wert. Üblicherweise wird die Säure bzw. Base in einem Anteil von 0,0001 bis 20 %, bevorzugt 0,001 bis 10 % verwendet.

Die Säuren können mit einem Anteil von 0,0001 bis 20 %, bevorzugt 0,01 bis 10 % verwendet werden.

Die erfindungsgemäßen Arzneimittel können hergestellt werden, indem das Vitamin in dem Solvens dispergiert wird. Das Vitamin kann direkt oder zur quantitativen Überführung durch Verwendung einer Stammlösung in dem Solvens dispergiert werden. Als Stammlösung können außer dem eigentlichen Solvens auch andere Solventien verwendet werden. Gegebenenfalls wird das pharmakologisch verträgliche Phosphonsäurederivat ebenfalls im Solvens dispergiert. Das Butanol oder Gemische von Butanol und Solvens werden ergänzt. Kosolventien sowie weitere Inhaltsstoffe wie z.B. Antioxidantien können bereits dem Solvens zugegeben oder später zugemischt werden. Der pH-Wert wird eingestellt, z.B. mit einer Base. Zum Schutz des Vitamins B₁₂ können Teile der Herstellung und Abfüllung der Lösungen unter Schutzgasatmosphäre, z.B. Stickstoffbegasung stattfinden.

Alternativ können Butanol und gegebenenfalls auch das pharmakologisch verträgliche Phosphonsäurederivat zunächst im Solvens gelöst werden und anschließend erst das Vitamin B₁₂ ergänzt werden.

Die erfindungsgemäßen pharmazeutischen Zubereitungen eignen sich generell für die Anwendung bei Mensch und Tier. Bevorzugt werden sie in der Tierhaltung und Tierzucht bei Nutz-, Zucht-, Zoo-, Labor-, Versuchs- und Hobbytieren eingesetzt.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär sowie Vögel wie z.B. Wachteln, Hühner, Gänse, Puten, Enten, Tauben und Vogelarten für Heim- und Zoohaltung.

Zu Labor- und Versuchstieren gehören Mäuse, Ratten, Meerschweinchen, Goldhamster, Kaninchen, Affen, Hunde und Katzen.

Zu den Hobbytieren gehören Kaninchen, Hamster, Ratten, Meerschweinchen, Mäuse, Pferde, Reptilien, entsprechende Vogelarten, Hunde und Katzen.

Weiterhin seien Fische genannt, und zwar Nutz-, Zucht-, Aquarien- und Zierfische aller Altersstufen, die in Süß- und Salzwasser leben.

Bei Hobbytieren werden die erfindungsgemäßen Zubereitungen bevorzugt bei Pferden, Kaninchen, Katzen und Hunden eingesetzt. Insbesondere eignen sie sich für die Anwendung bei Katzen und Hunden.

Bei Nutztieren werden die erfindungsgemäßen Zubereitungen bevorzugt bei Rind, Schaf, Schwein, Ziege, Pute und Huhn eingesetzt. Besonders bevorzugte Nutztiere sind Rind und Schwein.

Die Anwendung kann sowohl prophylaktisch, metaphylaktisch als auch therapeutisch erfolgen.

Erfindungsgemäße flüssige Formulierungen sind bevorzugt Emulsionen oder insbesondere Lösungen.

Die hier beschriebenen Formulierungen können auf verschiedene Arten dem Zielorganismus (Mensch oder Tier) zugeführt werden. Sie können beispielsweise parenteral, insbesondere durch Injektion (z. B. subkutan, intramuskulär, intravenös, intramammär, intraperitoneal), dermal, oral, rectal, vaginal oder nasal appliziert werden, wobei die orale und die parenterale Applikation - insbesondere durch Injektion - bevorzugt ist. Besonders bevorzugt ist die parenterale Applikation durch Injektion.

Die Verwendung mit den genannten Substanzen führt zu Arzneimitteln mit guter Stabilität des Vitamin B₁₂, insbesondere gegenüber Licht.

### Beispiele

Die Formulierungen der folgenden Beispiele werden hergestellt, indem die angegebenen Einsatzstoffe in Aqua pro Injektione oder Aqua destillata oder Aqua demineralisata gemischt oder gelöst werden. Zum quantitativen Einbringen des Cyanocobalamins in den Hauptansatz empfiehlt es sich, eine Stammlösung zu verwenden. Dies ist aber nicht zwingend erforderlich. Der pH-Wert der Lösungen kann durch Zugabe von Säuren oder Basen eingestellt werden. Die Lösungen werden unter Stickstoffschutz hergestellt und abgefühlt. Die Lösungen zur Injektion werden sterilfiltriert und in geeignete Behältnisse überführt. Prozentangaben in Gewichtsprozent beziehen sich auf das Gesamtvolumen des fertigen Präparats, (M/V).

### Beispiel 1

10 % Butaphosphan
0,005 % Cyanocobalamin (Vitamin B₁₂)
3,0 % n-Butanol
quantum satis Natriumhydroxid
ad 100 % Aqua pro Injektione

0,0005 g Vitamin B₁₂ werden in einer Teilmenge Aqua pro Injektione in der Wärme gelöst. 1,0 g Butaphosphan und 0,3 g n-Butanol werden in Aqua pro Injektione gelöst und die Stammlösung mit Cyanocobalamin wird ergänzt. Der pH-Wert wird mit Natriumhydroxid auf 5,6 (+/- 0,2) eingestellt und das Endgewicht wird mit Aqua pro Injektione zu 10 ml eingestellt.

### Beispiel 2

0,005 % Cyanocobalamin (Vitamin B₁₂)
3,0 % n-Butanol
quantum satis Natriumhydroxid
ad 100 % Aqua pro Injektion

0,0010 g Vitamin B₁₂ werden in einer Teilmenge Aqua pro Injektione in der Wärme gelöst. 0,6 g n-Butanol werden in Aqua pro Injektione gelöst und die Stammlösung mit Cyanocobalamin wird ergänzt. Der pH-Wert wird mit Natriumhydroxid auf 5,6 (+/- 0,2) eingestellt und das Endgewicht wird mit Aqua pro Injektione zu 20 ml eingestellt.

### Beispiel 3

10 % Butaphosphan
0,005 % Cyanocobalamin (Vitamin B₁₂)
2,0 % n-Butanol
quantum satis Natriumhydroxid
ad 100 % Aqua pro Injektione

0,0025 g Vitamin B₁₂ werden in einer Teilmenge Aqua pro Injektione in der Wärme gelöst. 5,0 g Butaphosphan und 1,0 g n-Butanol werden in Aqua pro Injektione gelöst und die Stammlösung mit Cyanocobalamin wird ergänzt. Der pH-Wert wird mit Natriumhydroxid auf 5,6 (+/- 0,2) eingestellt und das Endgewicht wird mit Aqua pro Injektione zu 50 ml eingestellt.

### Beispiel 4

20 % Butaphosphan
0,010 % Cyanocobalamin (Vitamin B₁₂)
3,0 % n-Butanol
quantum satis Kaliumhydroxid
ad 100 % Aqua pro Injektione

0,0010 g Vitamin B₁₂ werden in einer Teilmenge Aqua pro Injektione in der Wärme gelöst. 2,0 g Butaphosphan und 0,3 g n-Butanol werden in Aqua pro Injektione gelöst und die Stammlösung mit Cyanocobalamin wird ergänzt. Der pH-Wert wird auf 5,6 (+/- 0,2) eingestellt und das Endgewicht wird mit Aqua pro Injektione zu 10 ml eingestellt.

### Beispiel 5

10 % Butaphosphan
0,005 % Cyanocobalamin (Vitamin B₁₂)
4,0 % n-Butanol
quantum satis Natriumhydroxid
ad 100 % Aqua pro Injektion

0,005 g Vitamin B₁₂ werden in einer Teilmenge Aqua pro Injektione in der Wärme gelöst. 10,0 g Butaphosphan und 4,0 g n-Butanol werden in Aqua pro Injektione gelöst und die Stammlösung mit Cyanocobalamin wird ergänzt. Der pH-Wert wird mit Natriumhydroxid auf 5,6 (+/- 0,2) eingestellt und das Endgewicht wird mit Aqua pro Injektione zu 100 ml eingestellt.

### Beispiel 6

15 % Butaphosphan
0,0075 % Cyanocobalamin (Vitamin B₁₂)
3,0 % n-Butanol
quantum satis Meglumin
ad 100 % Aqua pro Injektione

0,00075 g Vitamin B₁₂ werden in einer Teilmenge Aqua pro Injektione in der Wärme gelöst. 1,5 g Butaphosphan und 0,3 g n-Butanol werden in Aqua pro Injektione gelöst und die Stammlösung mit Cyanocobalamin wird ergänzt. Der pH-Wert wird mit Meglumin auf 5,6 (+/- 0,2) eingestellt und das Endgewicht wird mit Aqua pro Injektione zu 10 ml eingestellt.

### Beispiel 7

30 % Butaphosphan
0,015 % Cyanocobalamin (Vitamin B₁₂)
3,0 % n-Butanol
quantum satis Arginin
ad 100 % Aqua pro Injektione

0,0015 g Vitamin B₁₂ werden in einer Teilmenge Aqua pro Injektione in der Wärme gelöst. 3,0 g Butaphosphan und 0,3 g n-Butanol werden in Aqua pro Injektione gelöst und die Stammlösung mit Cyanocobalantin wird ergänzt. Der pH-Wert wird mit Arginin auf 5,6 (+/- 0,2) eingestellt und das Endgewicht wird mit Aqua pro Injektione zu 10 ml eingestellt.

### Beispiel 8

0,010 % Cyanocobalamin (Vitamin B₁₂)
3,0 % n-Butanol
quantum satis Arginin
ad 100 % Aqua pro Injektione

0,0010 g Vitamin B₁₂ werden in einer Teilmenge Aqua pro Injektione in der Wärme gelöst. 0,3 g n-Butanol werden mit Aqua pro Injektione gemischt und die Stammlösung mit Cyanocobalamin wird ergänzt. Der pH-Wert wird mit Arginin auf 5,6 (+/- 0,2) eingestellt und das Endgewicht wird mit Aqua pro Injektione zu 10 ml eingestellt.

### Stabilität gegenüber Licht

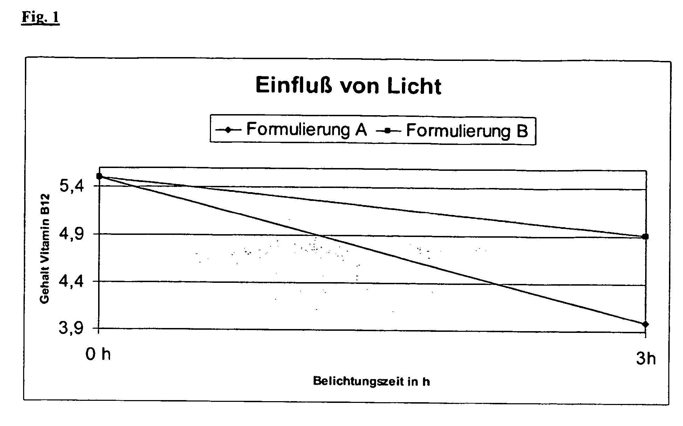

Die hier beschriebenen Formulierungen haben eine verbesserte Lichstabilität gegenüber anderen Formulierungen gezeigt.. In der nachfolgenden Tabelle sind ausgewählte Beispiele dazu aufgelistet. In der Fig. 1 sind die Ergebnisse nochmals graphisch dargestellt. (Fig. 1: Vergleich der Lichtstabilitäten einer Formulierung ohne n-Butanol [A] gegen eine Formulierung mit n-Butanol [B, gemäß Beispiel 1]).

**Tabelle 1: Vergleich der Lichtstabilitäten einer Formulierung ohne n-Butanol (A) gegen eine Formulierung mit n-Butanol (B).**

| | Anfangsgehalt Vitamin B₁₂ in mg/100ml | Gehalt an Vitamin B₁₂ in mg/100ml nach 3 h Belichtung |
|---|---|---|
| Formulierung A | 5,50 | 3,99 |
| Formulierung B gemäß Beispiel 1 | 5,53 | 4,91 |

## Patentansprüche

1. Zubereitung enthaltend Vitamin B₁₂ und Butanol sowie weiterhin ein pharmakologisch verträgliches Phosphonsäurederivat.

2. Zubereitung gemäß Anspruch 1, **dadurch gekennzeichnet, dass** sie flüssig ist.

3. Zubereitung gemäß einem der vorstehenden Ansprüche, **dadurch gekennzeichnet, dass** das Butanol ausgewählt ist aus: n-Butanol oder iso-Butanol.

4. Zubereitung gemäß einem der vorstehenden Ansprüche enthaltend n-Butanol.

5. Zubereitung gemäß einem der Ansprüche 1 bis 4, enthaltend ein pharmakologisch verträgliches Phosphonsäurederivat ausgewählt aus Butaphosphan und Toldimfos.

6. Zubereitung gemäß Anspruch 5, enthaltend das pharmakologisch verträgliche Phosphonsäurederivat Butaphosphan.

7. Verwendung einer Zubereitung gemäß einem der vorstehenden Ansprüche zur Herstellung eines Arzneimittels.

8. Verwendung von Butanol zur Stabilisierung von Vitamin B₁₂.

9. Verwendung von Butanol zur Herstellung von Zubereitungen des Vitamin B₁₂ mit verbesserter Vitamin B₁₂-Stabilität.

## Claims

1. Preparation comprising vitamin B₁₂ and butanol and additionally a pharmacologically acceptable phosphonic acid derivative.

2. Preparation according to Claim 1, **characterized in that** it is liquid.

3. Preparation according to either of the preceding claims, **characterized in that** the butanol is selected from: n-butanol or isobutanol.

4. Preparation according to any of the preceding claims comprising n-butanol.

5. Preparation according to any of Claims 1 to 4, comprising a pharmacologically acceptable phosphonic acid derivative selected from butaphosphan and toldimfos.

6. Preparation according to Claim 5, comprising the pharmacologically acceptable phosphonic acid derivative butaphosphan.

7. Use of a preparation according to any of the preceding claims for the manufacture of a medicament.

8. Use of butanol for stabilizing vitamin B₁₂.

9. Use of butanol for producing preparations of vitamin B₁₂ with improved vitamin B₁₂ stability.

## Revendications

1. Composition contenant de la vitamine B₁₂ et du butanol ainsi qu'en outre un dérivé pharmacologiquement acceptable de l'acide phosphonique.

2. Composition selon la revendication 1, **caractérisée en ce qu'**elle est liquide.

3. Composition selon l'une quelconque des revendications précédentes, **caractérisée en ce que** le butanol est choisi parmi : le n-butanol ou l'iso-butanol.

4. Composition selon l'une quelconque des revendications précédentes, contenant du n-butanol.

5. Composition selon l'une quelconque des revendications 1 à 4, contenant un dérivé pharmacologiquement acceptable de l'acide phosphonique choisi parmi le butaphosphan et le toldimfos.

6. Composition selon la revendication 5, contenant le dérivé pharmacologiquement acceptable de l'acide phosphonique butaphosphan.

7. Utilisation d'une composition selon l'une quelconque des revendications précédentes pour la préparation d'un médicament.

8. Utilisation de butanol pour la stabilisation de la vitamine B₁₂.

9. Utilisation de butanol pour la préparation de compositions de la vitamine B₁₂ présentant une stabilité améliorée de la vitamine B₁₂.
